(19) Europäisches Patentamt
European Patent Office
Office européen des brevets

(11) EP 4 583 931 B1

(12) **EUROPEAN PATENT SPECIFICATION**

(45) Date of publication and mention of the grant of the patent:
**15.04.2026 Bulletin 2026/16**

(21) Application number: **24775993.9**

(22) Date of filing: **23.08.2024**

(51) International Patent Classification (IPC):
***A61L 27/54*** *(2006.01)* ***C08L 83/04*** *(2006.01)*

(52) Cooperative Patent Classification (CPC):
(C-Sets available)
**C08L 83/04; A61L 24/0015; A61L 24/046;**
A61L 2300/202; A61L 2300/404; C08G 77/12;
C08G 77/20 (Cont.)

(86) International application number:
**PCT/IB2024/058218**

(87) International publication number:
**WO 2025/041093 (27.02.2025 Gazette 2025/09)**

(54) **LOW TEMPERATURE-CURABLE LIQUID SILICONE RUBBER CARRIER FOR ACTIVE PHARMACEUTICAL INGREDIENT**

BEI NIEDRIGER TEMPERATUR HÄRTBARER FLÜSSIGER SILIKONKAUTSCHUKTRÄGER FÜR EINEN PHARMAZEUTISCHEN WIRKSTOFF

SUPPORT EN CAOUTCHOUC DE SILICONE LIQUIDE DURCISSABLE À BASSE TEMPÉRATURE POUR INGRÉDIENT PHARMACEUTIQUE ACTIF

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.08.2023 US 202318454263**

(43) Date of publication of application:
**16.07.2025 Bulletin 2025/29**

(73) Proprietor: **Cilag GmbH International**
**6300 Zug (CH)**

(72) Inventor: **OU, Duan Li**
**Raritan, New Jersey 08869 (US)**

(74) Representative: **Carpmaels & Ransford LLP**
**One Southampton Row**
**London WC1B 5HA (GB)**

(56) References cited:
**US-A1- 2021 369 276**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**(Cont. next page)**

(52) Cooperative Patent Classification (CPC): (Cont.)

C-Sets
**A61L 24/046, C08L 83/04;**
**C08L 83/04, C08L 83/00, C08K 3/36, C08K 5/56;**
**C08L 83/04, C08L 83/00, C08K 5/56;**
**C08L 83/04, C08L 83/00, C08K 9/06, C08K 5/56**

## Description

BACKGROUND

A wound closure system (also referred to as a skin closure system) may be used at the conclusion of a surgical procedure on a patient to close a wound (e.g., a surgical incision) that was formed in the patient's skin for accessing a target anatomical structure. By way of example, wound closure systems may include components such as sutures, substrates, and/or liquid topical skin adhesives that are applied by a surgeon to approximate the edges of the wound and, in some cases, form a stable and protective layer over the wound that promotes efficient healing. In some instances, one or more components of the applied wound closure system may be absorbed by the patient during the healing process. Following healing of the wound, remaining components of the wound closure system may be removed from the skin by a surgeon, and/or they may automatically separate from the skin such that they may be discarded by the patient.

While various wound closure systems and associated components and methods have been made and used, it is believed that no one prior to the inventors has made or used the invention described in the appended claims.

BRIEF DESCRIPTION OF THE DRAWINGS

The accompanying drawings, which are incorporated in and constitute a part of this specification, illustrate embodiments of the invention, and, together with the description given below, serve to explain the principles of the present invention.

FIG. 1 depicts a perspective view of an example of a wound closure system that includes a wound closure device, an adhesive applicator, and an adhesive spreader;

FIG. 2 depicts a disassembled perspective view of the wound closure device of FIG. 1, showing a mesh layer, a pressure sensitive adhesive layer, and a removable backing layer;

FIG. 3A depicts a perspective view of the wound closure device of FIG. 1 aligned longitudinally with a wound in the skin of a patient, showing a central section of the backing layer having been removed;

FIG. 3B depicts a perspective view of the wound closure device of FIG. 1 applied to the patient's skin over the wound to approximate the edges of the wound;

FIG. 3C depicts a perspective view of the wound closure device of FIG. 1 applied to the patient's skin over the wound, showing remaining sections of the backing layer having been removed so the wound closure device is fully adhered to the skin;

FIG. 3D depicts a perspective view of the wound closure device of FIG. 1 applied to the patient's skin over the wound, showing application of a liquid topical skin adhesive onto the mesh layer with the adhesive applicator;

FIG. 3E depicts a perspective view of the wound closure device of FIG. 1 applied to the patient's skin over the wound, showing the adhesive spreader positioned against the patient at the start of an adhesive spreading stroke for spreading the applied topical skin adhesive over and through the wound closure device;

FIG. 3F depicts a perspective view of the wound closure device of FIG. 1 applied to the patient's skin over the wound, showing the adhesive spreader during a subsequent portion of the adhesive spreading stroke; and

FIG. 4 depicts a chart showing the results of nuclear magnetic resonance ("NMR") spectroscopy for a low-temperature-cured liquid silicone rubber.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "side," "upwardly," and "downwardly" also are used herein for reference to relative positions and directions. Such terms are used below with reference to views as illustrated for clarity and are not intended to limit the invention described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose described herein.

I. Wound Closure System

FIG. 1 shows an example of a wound closure system (10) that includes a wound closure device (20) (also referred to as a patch) configured to be applied to a patient's wound (W) (see FIG. 3A), an adhesive applicator (40) configured to apply a topical skin adhesive (54) (see FIG. 3D) to the applied wound closure device (20), and an adhesive spreader (60) configured to spread the applied topical skin adhesive (54) onto and through wound closure device (20). Each of these components is described in greater detail below.

As shown best in FIG. 2, wound closure device (20) of the present version has an elongate, generally rectangular shape and a triple layer construction. More specifically, wound closure device (20) includes a layer of substrate in the form of a textile mesh (22), a layer of pressure sensitive adhesive (24) formed as a continuous or non-continuous coating along the lower skin-facing side of mesh (22), and a layer of backing (26) removably applied to the lower side of pressure sensitive adhesive (24). It will be understood that the term "wound closure device" as used herein encompasses wound closure device (20) both with and without backing (26), which may be removed and discarded during application of wound closure device (20) to a patient, as described in greater detail below.

Mesh (22) is a flexible and porous biocompatible substrate and can be configured to retain a liquid topical skin adhesive. Mesh (22) may be formed of polyethylene terephthalate (PET) or any other suitable surgical textile material, but it will be appreciated that mesh can be a porous film, strip, or tape formed from polymeric materials (e.g., polymeric films or tapes) by extrusion, perforation, cutting, and the like, or alternatively, mesh can be a textile woven or non-woven fabric. Pressure sensitive adhesive (24) is configured to enable wound closure device (20) to self-adhere to a patient's skin in response to a pressure being applied to the upper side of mesh (22) during its application by a surgeon. Backing (26) serves to protect pressure sensitive adhesive (24) before application of wound closure device (20) to the patient. In the present version, backing (26) includes elongate arrays of perforations that extend longitudinally and define an elongate central backing section (28) and a pair of elongate side backing sections (30). Though each backing section (28, 30) is shown as generally rectangular in the present version, backing sections (28, 30) may be of various alternative shapes, sizes, and quantities in other versions.

As shown in FIG. 1, adhesive applicator (40) includes an applicator body (42), a plunger unit (44) slidably received within an open proximal end of applicator body (42), and a static mixer (46) secured to a distal end of applicator body (42). Applicator body (42) includes a pair of barrels (48) arranged side by side, where each barrel (48) houses a respective part of a two-part liquid topical skin adhesive. Plunger unit (44) includes a pair of plungers (50) that are arranged side by side and are interconnected at their proximal ends by a bridge (52). Each plunger (50) is actuatable distally through a respective barrel (48) of applicator body (42) to force the corresponding liquid adhesive part distally into static mixer (46). Static mixer (46) is configured to receive the first and second adhesive parts and direct them around and through a series of static baffles and passages (not shown) that mix the two adhesive parts together into a homogenous liquid adhesive (54) that is then dispensed through an open distal end of static mixer (46), as shown in FIG. 3D described below.

In the present version, liquid topical skin adhesive (54) is in the form of a silicone-based topical skin adhesive that is configured to cure on skin at body temperature in less than two minutes. Once cured, topical skin adhesive (54) remains elastomeric such that a given section of cured adhesive (54) is configured to stretch up to 160% of its cured length and then fully recover to the cured length. Accordingly, wound closure system (10) may be particularly effective for use on actuatable

body parts of a patient such as a knee, wrist, elbow, or other joint, for example.

As also shown in FIG. 1, adhesive spreader (60) of the present version has a monolithic body that includes a proximal body portion (62) configured to be gripped by a user, a distal body portion (64) that terminates at a tip configured to spread applied topical skin adhesive (54), and an intermediate flexural body portion (66) that interconnects the proximal and distal body portions (62, 64). Flexural body portion (66) is configured to elastically deform so that distal body portion (64) angularly deflects relative to proximal body portion (62) to promote effective spreading of topical skin adhesive (54) along wound closure device (20) with a suitable normal force within a predetermined range.

FIGS. 3A-3D show an example of wound closure system (10) being used to close a wound (W) formed in the skin (S) of a patient. In some procedures, wound (W) may be at least partially closed with one or more sutures, for example at deeper portions of wound (W), prior to use of wound closure system (10). Additionally, before wound closure device (20) is applied to the patient, it may be trimmed by a surgeon to any suitable shape as desired.

As shown in FIG. 3A, central backing section (28) is removed from wound closure device (20) to expose a central window of mesh (22) and pressure sensitive adhesive (24), and an imaginary centerline of wound closure device (20) is aligned longitudinally with the edges of wound (W). Wound closure device (20) is then applied to the patient skin (S) over wound (W) and the surgeon applies pressure to the central portion of mesh (22) to force pressure sensitive adhesive (24) to adhere to the skin (S), thus fixing the edges of wound (W) relative to one another. Before this step, the edges of wound (W) may be held in an approximated state by the surgeon. Alternatively, during this step the central portion of wound closure device (20) may be applied in a laterally alternating manner to approximate the edges of wound (W) during application. With either approach, wound closure device (20) is configured to hold the edges of wound (W) in an approximated state following this initial step of application. Once the surgeon is satisfied with the position of wound closure device (20) relative to wound (W), the surgeon may then remove the remaining two side backing sections (30) to adhere the reminder of wound closure device (20) to skin (S).

As shown in FIG. 3D, adhesive applicator (40) is then used to apply a pattern of topical skin adhesive (54) to the upper side of mesh (22) of the applied wound closure device (20). While adhesive applicator (40) is shown applying a linear bead of topical skin adhesive (54) in the present version, it will be appreciated that various other patterns of topical skin adhesive (54) may be applied in other versions, such as a T-shaped pattern or a wave-shaped pattern, as described in greater detail below.

As shown in FIGS. 3E-3F, adhesive spreader (60) is then used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). In that regard, the layers of mesh (22) and pressure sensitive adhesive (24) may be at least partially permeable to permit forced passage of topical skin adhesive (54) therethrough. As shown in FIG. 3E, flexural body portion (66) of adhesive spreader (60) may be in a non-deformed state when adhesive spreader (60) is first positioned against wound closure device (20) at the beginning of an adhesive spreading stroke. As adhesive spreader (60) is dragged longitudinally along wound closure device (20), the input force exerted by the user may cause flexural body portion (66) to elastically deform such that distal body portion (64) angularly deflects relative to proximal body portion (62), as shown in FIG. 3F. The degree of deformation of flexural body portion (66) may be directly related to the viscosity of topical skin adhesive (54). In particular, a greater viscosity may require that the user exert a greater input force through proximal body portion (62) to effectively spread topical skin adhesive (54) over and through wound closure device (20), such that the flexural body portion (66) deforms a relatively greater amount. Conversely, a lesser viscosity may require a lesser input force such that the flexural body portion (66) deforms less or not at all.

Optionally, topical skin adhesive (54) may also be spread over adjacent portions of skin (S) not covered by wound closure device (20) to ensure that an entirety of mesh (22) is embedded with topical skin adhesive (54). For instance, and by way of example only, topical skin adhesive (54) may be spread onto at least 1 cm of skin (S) about the entire outer perimeter of the applied wound closure device (20). Once topical skin adhesive (54) has been fully spread over wound closure device (20), any topical skin adhesive (54) on skin (S) beyond the perimeter of device (20) may then be wiped away with sterile gauze, for example. Additionally, in some instances, a quantity of topical skin adhesive (54) may be applied between the edges of wound (W) before wound closure device (20) is applied to the skin (S). The applied topical skin adhesive (54) is then cured within and over wound closure device (20) to form a composite microbial barrier over wound (W) that maintains a protective environment that promotes efficient healing. Following healing of wound (W), wound closure device (20) may be removed from the skin (S) manually (e.g., by a surgeon) or it may automatically separate from the skin (S) such that it may be discarded by the patient.

Wound closure system (10) may be further configured and operable in accordance with at least some of the teachings of U.S. Pat. Pub. No. 2021/0369258, entitled "Systems, Devices and Methods for Dispensing and Curing Silicone Based Topical Skin Adhesives," published December 2, 2021; U.S. Pat. Pub. No. 2021/0371190, entitled "Systems, Methods and Devices for Aerosol Spraying of Silicone Based Topical Skin Adhesives for Sealing Wounds," published December 2, 2021; U.S. Pat. Pub. No. 2021/0371658, entitled "Novel Topical Skin Closure Compositions and Systems," published December 2, 2021; U.S. Pat. Pub. No. 2021/0369639, entitled "Novel Antimicrobial Topical Skin

Closure Compositions and Systems," published December 2, 2021; U.S. Pat. Pub. No. 2021/0369276, entitled "Anisotropic Wound Closure Systems," published December 2, 2021; U.S. Pat. App. No. 17/667,950, entitled "Gas Sterilizable Syringes Having Apertures Covered by Gas Permeable Barriers for Enabling Ingress and Egress of Sterilization Gases While Preventing Leakage of Flowable Materials," filed on February 9, 2022; U.S. 17/991,992, entitled "Application of Topical Skin Adhesive to Surgical Mesh," filed on November 22, 2022; U.S. 17,991,945, entitled "Surgical Mesh Securing Device For Wound Closure System," filed on November 22, 2022; and/or U.S. 17/991,950, entitled "Wound Closure System Having Microcannulaic Pathways," filed on November 22, 2022.

II. Illustrative Low-Temperature-Cured Liquid Silicone Rubber Carriers

As mentioned above, wound closure device (20) is configured to be applied to a patient's wound (W) while topical skin adhesive (54) (e.g., a silicone-based topical skin adhesive) is configured to cure within and over wound closure device (20). In some instances, it may be desirable to apply an active pharmaceutical ingredient (i.e., an "API") directly onto and/or into the wound (W) while wound closure device (20) and topical skin adhesive (54) form the composite microbial barrier over wound (W). Silicon-based materials, like those used for the topical skin adhesive (54), can be effective carriers for an API. Such silicone-based materials can be formed from liquid silicone rubber. However, conventional liquid silicone rubbers are manufactured in large scale production equipment (e.g., in an injection molding process) and processed at high temperatures (e.g., at 190 °C). APIs typically decompose at such high temperatures, and thus, a liquid silicone rubber used to carry an API must be processable at lower temperatures. Therefore, it may be desirable to utilize a liquid silicone rubber carrier for an API that can be cured at low temperatures (i.e., 100 °C or less) and incorporated into a wound closure device, such as wound closure device (20).

An illustrative low-temperature-curable liquid silicone rubber can include a polydimethylsiloxane, a crosslinking agent, a silica filler, and a platinum tetramethyldivinyl disiloxane vinyl acetate catalyst. Not only can such liquid silicone rubbers be cured at relatively low temperatures (e.g., less than 100 °C), the low-temperature-cured liquid silicone rubbers as described herein can exhibit mechanical properties that are comparable to conventional liquid silicone rubbers.

In an illustrative version of the low-temperature-curable liquid silicone rubber, the polydimethylsiloxane can be crosslinkable. A suitable example of a crosslinkable polydimethylsiloxane can be vinyl-terminated polydimethylsiloxane, as represented below by Formula (I), but it will be appreciated that one or more other types of crosslinkable polydimethylsiloxane can be employed in a low-temperature-curable liquid silicone rubber. In some examples, the vinyl-terminated polydimethylsiloxane can have a number average molecular weight between about 10,000 and about 500,000; or in some examples, between about 25,000 and about 120,000 (i.e., for low-molecular-weight vinyl-terminated polydimethylsiloxane). In certain examples, the low-temperature-curable liquid silicone rubber can comprise about 60% to about 90%, by weight of the low-temperature-curable liquid silicone rubber, of the polydimethylsiloxane.

Formula (I):

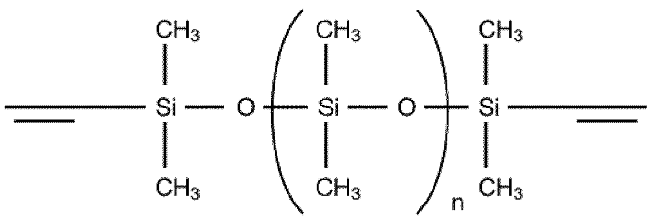

As defined herein, unless stated otherwise, number average molecular weight can be determined based on the relationship between kinematic viscosity and molecular weight (page 11, SILICONE FLUIDS: STABLE, INERT MEDIA ENGINEERING AND DESIGN PROPERTIES, Catalog published by Gelest, Inc. 11 East Steel Rd. Morrisville, Pa. 19067). Using A. J. Barry's relationship for molecular weights (M) >2,500 correlating the kinematic viscosity μ expressed in centistokes (cSt) at 25 °C, the molecular weight M of silicones can be estimated as follows:

$$\log \mu_{cSt} = 1.00 + 0.0123\ M^{0.5}$$

(as published by A. J. Barry in the Journal of Applied Physics 17, 1020 (1946))

The low-temperature-curable liquid silicone rubber can further include a reinforcing component or filler to provide for enhanced mechanical strength. The reinforcing filler can be a silica filler (e.g., silica nanoparticles), where -OH groups on the surface thereof can form hydrogen bonds with the oxygen bridge of the Si-O-Si linkage of the crosslinked polydimethylsiloxane. In some examples, the silica filler can already be mixed with the polydimethylsiloxane. The silica

filler may be surface-treated (e.g., hexamethyl silyl surface treatment) to allow for compatibility with the crosslinkable polydimethylsiloxane and prevent phase separation. A suitable example of a surface-treated silica filler can be hexamethyldisilazane-treated silica, but it will be appreciated that other types of surface-treated silica fillers can be employed in a low-temperature-curable liquid silicone rubber. In some examples, the surface-treated silica is surface-treated fumed silica such as hexamethyldisilazane-treated fumed silica. In certain examples, the low-temperature-curable liquid silicone rubber can comprise about 10% to about 40%, by weight of the low-temperature-curable liquid silicone rubber, of the surface-treated silica filler. In another example, the low-temperature-curable liquid silicone rubber can include a commercially available filler-reinforced polydimethylsiloxane. In one such version, a suitable filler-reinforced polydimethylsiloxane is prepared by mixing silica filler with vinyl-terminated polydimethylsiloxane while performing surface treatment of filler.

Suitable crosslinking agents for the illustrative low-temperature-curable liquid silicone rubber can include, but are not limited to, one or more of polymethylhydrosiloxane and polymethylhydro-co-polydimethylsiloxane. Polymethylhydrosiloxane, as represented below by Formula (II), can have, in certain examples, a number average molecular weight between about 1,000 and about 3,000; or in certain examples, about 1,400 and about 2,100. Polymethylhydro-co-polydimethylsiloxane, as represented below by Formula (III), can have, in certain examples, a number average molecular weight between about 900 and about 5,000.

Formula (II):

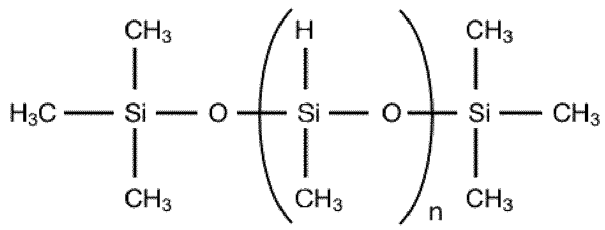

Formula (III):

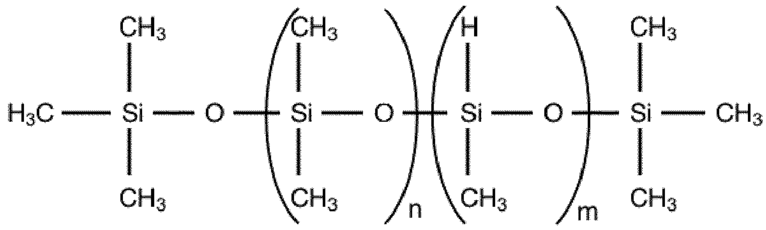

Illustrative low-temperature-curable liquid silicone rubbers can include a platinum tetramethyldivinyl disiloxane vinyl acetate catalyst. The platinum tetramethyldivinyl disiloxane vinyl acetate catalyst can be formed by reacting a platinum tetramethyldivinyl disiloxane (e.g., Karstedt's catalyst) with vinyl acetate as represented below in Scheme 1. In certain examples, the reaction time to form the catalyst can be three hours or more; in certain examples, four hours or more. In certain examples, the low-temperature-curable liquid silicone rubber can comprise about 0.025% to about 0.1%, by weight of the low-temperature-curable liquid silicone rubber, of the platinum tetramethyldivinyl disiloxane vinyl acetate catalyst; in certain examples about 0.04% to about 0.09%, by weight of the low-temperature-curable liquid silicone rubber, of the platinum tetramethyldivinyl disiloxane vinyl acetate catalyst; and in certain examples about 0.05% to about 0.075%, by weight of the low-temperature-curable liquid silicone rubber, of the platinum tetramethyldivinyl disiloxane vinyl acetate catalyst. The platinum tetramethyldivinyl disiloxane and vinyl acetate in the catalyst can be provided in a mole ratio of 2 to 100 of vinyl acetate to platinum tetramethyldivinyl disiloxane. In certain examples, the vinyl acetate can comprise less than 50%, by weight, of the platinum tetramethyldivinyl disiloxane vinyl acetate catalyst.

Scheme (I):

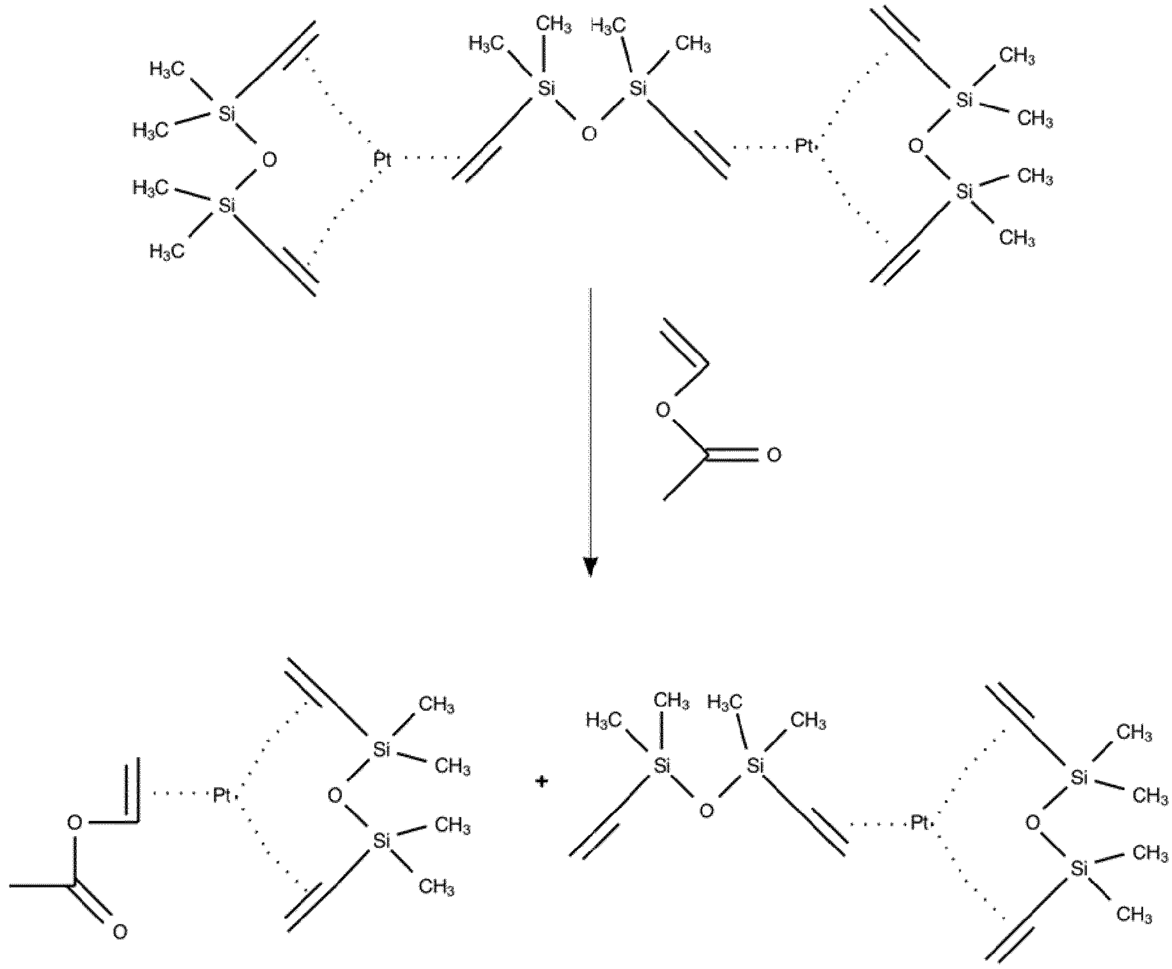

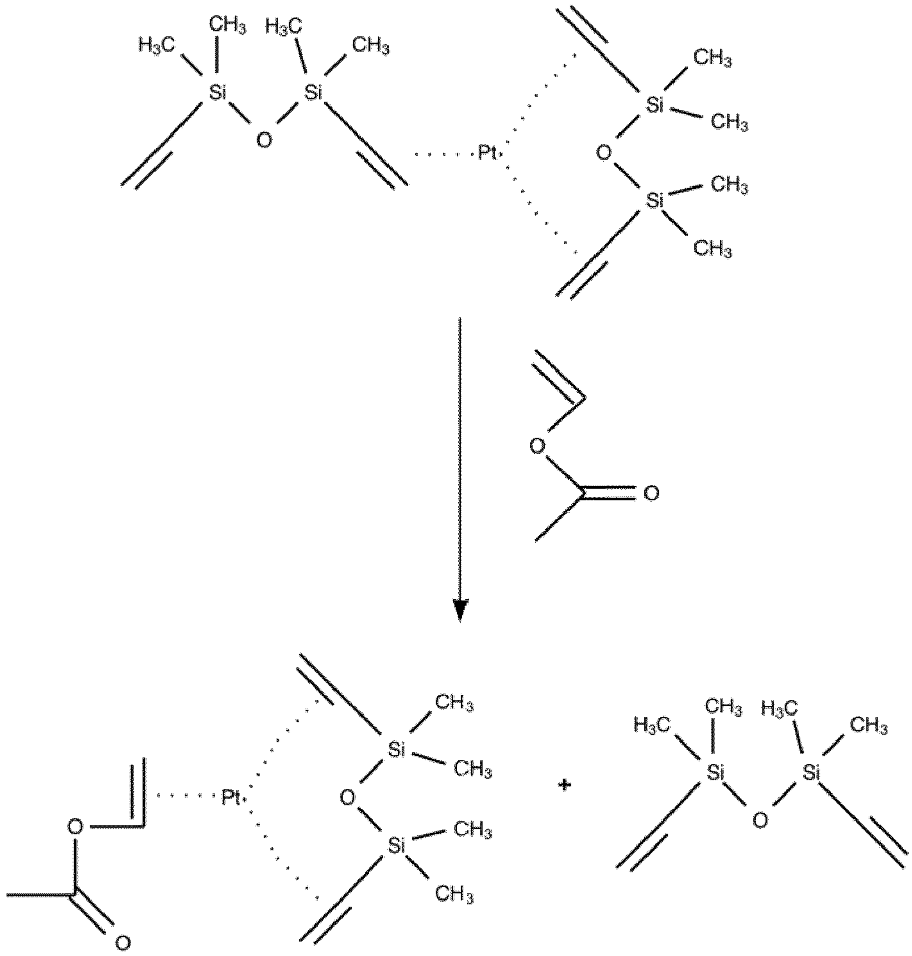

In certain examples, the platinum tetramethyldivinyl disiloxane vinyl acetate catalyst can be the primary catalyst. In such examples, the low-temperature-curable liquid silicone rubber can include one or more co-catalysts, where the platinum tetramethyldivinyl disiloxane vinyl acetate catalyst, as the primary catalyst, can be the main driving force for a reaction. In other examples, the platinum tetramethyldivinyl disiloxane vinyl acetate catalyst can be substantially the only catalyst included in the low-temperature-curable liquid silicone rubber. In such examples, the platinum tetramethyldivinyl disiloxane vinyl acetate catalyst is the only catalyst intentionally included in the low-temperature-curable liquid silicone rubber, no other catalysts are intentionally added, and any other catalysts in the low-temperature-curable liquid silicone rubber are included in trace amounts (e.g., typically from about 0.0001% to about 0.0015% by weight of low-temperature-curable liquid silicone rubber).

In certain examples, the low-temperature-curable liquid silicone rubber can further include one or more APIs. As described herein, the API can transfer from the low-temperature-cured liquid silicone rubber to an intended recipient. In one example, the API can be triclosan. It will be appreciated, however, that any of a variety of APIs could be included in a low-temperature-curable liquid silicone rubber, including, but not limited to, peptide protein and enzymes, contraceptive hormones, antibacterial agents, antifungal agents, anti-inflammatory medication, mannitol, and atorvastatin. In certain examples, illustrative low-temperature-curable liquid silicone rubbers can comprise about 1% to about 50%; in certain examples, about 2% to about 40%; in certain examples, about 5% to about 35%; in certain examples, 5% to about 20%; in certain examples, about 5% to about 10%, by weight of the low-temperature-curable liquid silicone rubber, of the API.

The present low-temperature-curable liquid silicone rubber can be provided in a two-part kit. In certain examples, the two-part kit can include two equal parts, by weight (i.e., the first part and the second part of the kit have equal weight). The first part can include vinyl-terminated polydimethylsiloxane and surface-treated silica filler mixed with a platinum tetramethyldivinyl disiloxane vinyl acetate catalyst solution (1.17% Pt, by weight, in xylene). The second part can include vinyl-terminated polydimethylsiloxane and surface-treated silica filler mixed with a crosslinking agent. The second part may optionally include one or more APIs (e.g., triclosan). Each part can be mixed with a high-speed mixer.

An applicator, similar to the adhesive applicator (40) of FIG. 1, can facilitate the mixing and delivery of the two-part solution to provide a low-temperature-curable liquid silicone rubber. For example, the respective parts of the two-part kit can be loaded into neighboring barrels of the applicator to be mixed by a static mixer. In certain examples, the low-temperature-curable liquid silicone rubber can be injection molded into a mold. Further, in certain examples, the low-temperature-cured liquid silicone rubber can be integrated into a mesh for use within a wound closure system, such as, for example, the system disclosed in U.S. Pat. App. No [Atty. Ref. ETH6172USNP], entitled "Wound Closure System Having Medicant," filed on even date herewith. The illustrative low-temperature-curable liquid silicone rubber can be cured at a relatively low temperature (e.g., about 80 °C) at a cure time of about one minute or less. In certain examples, the illustrative low-temperature-curable liquid silicone rubber is cured at a temperature of about 60 °C to about 100 °C. In certain examples, the illustrative low-temperature-curable liquid silicone rubber is cured at about 80 °C. In certain examples, the illustrative low-temperature-curable liquid silicone rubber is cured in less than about 30 seconds.

Conventional liquid silicone rubbers can also be formed from a two-part kit, but many must be cured at relatively high temperatures (e.g., about 200 °C). The first part of such conventional liquid silicone rubbers include Karstedt's catalyst-and not the present platinum tetramethyldivinyl disiloxane vinyl acetate catalyst described herein. In such examples, polydimethylsiloxane reacts quickly with a crosslinking agent in the presence of Karstedt's catalyst. Vinyl acetate may be used in the second part of such conventional liquid silicone rubbers as an inhibitor to control the activation of Karstedt's catalyst. In this role, however, vinyl acetate is included in much greater amounts (e.g., a mole ratio of several hundreds to tens of thousands of vinyl acetate to platinum tetramethyldivinyl disiloxane) than described above for the illustrative platinum tetramethyldivinyl disiloxane vinyl acetate catalyst, which, when formed prior to combination with other liquid silicone rubber components, is its own catalyst, distinct from Karstedt's catalyst. For example, the illustrative platinum tetramethyldivinyl disiloxane vinyl acetate catalyst can be identified by NMR spectroscopy separately from Karstedt's catalyst, as illustrated by the NMR spectroscopy chart of FIG. 4. That is, while Karstedt's catalyst is known to register a $^{195}Pt$ signal at about -6143 ppm, the platinum tetramethyldivinyl disiloxane vinyl acetate catalyst registers $^{195}Pt$ signals at about -5837 ppm and -6143 ppm, as shown in FIG. 4.

Unless stated otherwise, $^{195}Pt$ NMR spectroscopy was performed at 30 °C under quantitative NMR acquisition conditions with inverse-gated broadband $^{1}H$-decoupling using a 400 MHz (9.4T) Agilent Technologies DD2 NMR spectrometer operating at 86 MHz. Typically, the NMR spectrometer is equipped with an Agilent OneNMR probe and controlled by VnmrJ 3.2 NMR system software. Chemical shift was referenced to potassium hexachloroplatinate at chemical shift $\delta$=0 ppm. NMR analysis was performed in benzene-$d_6$. Typically, to the catalyst sample submitted for $^{195}Pt$ NMR analysis, benzene-$d_6$ was added to a final concentration of 20% v/v.

There is also provided a method of forming a low-temperature-cured liquid silicone rubber, the method comprising: (a) combining a crosslinkable polydimethylsiloxane, a surface-treated silica filler, a crosslinking agent, and a platinum tetramethyldivinyl disiloxane vinyl acetate catalyst to form a reaction mixture; and (b) heating the reaction mixture to a temperature of about 60 °C to about 100 °C to form the low-temperature-cured liquid silicone rubber. In some examples, the reaction mixture further comprises one or more APIs.

## IV. Procedures for Preparing and Testing Example Specimens

Specimens were prepared for mechanical testing by coating a Teflon substrate with a 1 mm silicone film that is 203 mm (8 inches) by 38.1 mm (1.5 inches). The two-part liquid silicon rubber examples were applied to a substrate via a dual-barrel syringe, as described herein, and cured at a given temperature for one minute. The resulting silicone films were peeled from the substrate after curing.

### Tensile Strength Testing

The specimens were cut into dog-bone shapes in accordance with American Society for Testing and Materials ("ASTM)" D412: 12 mm wide with a 59-mm long narrow section. The testing constituted a slightly modified version of ASTM D412. A TJ-6 was used as the dual-column Instron Universal Tester and the 43.39 kg (100-lb) load cell was LC-18. The gauge length was set to 100 mm, the strain rate was set to 500 mm/min, and each specimen was preloaded to 0.100 N at a strain rate of 100 mm/min.

### Durometer Hardness Testing

The specimens were two overlaid pieces of the silicon film, each having a thickness of 1 mm and being cut to 127 mm (5 inches) by 38.1 mm (1.5 inches). The testing constituted a slightly modified version of ASTM D2240. A Rex shore M durometer gauge was used to measure five locations on the strip, where the result is the average of the five measurements.

## V. Examples

Unless stated otherwise, all % are % by weight.

Inventive Catalyst

An illustrative platinum tetramethyldivinyl disiloxane vinyl acetate catalyst was formed from reacting 30 g of a complex of 2.2% platinum tetramethyldivinyl disiloxane (i.e., Karstedt's catalyst) in xylene with 26.5 g vinyl acetate for three hours.

Inventive Example 1

An illustrative low-temperature-cured liquid silicone rubber was formed from a two-part kit. To form the first part of the kit, 97 g of a mixture of vinyl-terminated polydimethylsiloxane and fumed silica particles was combined with 3 g of the platinum tetramethyldivinyl disiloxane vinyl acetate catalyst of Inventive Catalyst in a 5% low-molecular-weight vinyl-terminated polydimethylsiloxane solution using a high-speed centrifugal mixer (i.e., FlackTek DAC150 FV-K) at 3470 rpm for 5 minutes. To form the second part of the kit, 97 g of a mixture of vinyl-terminated polydimethylsiloxane and fumed silica particles was combined with 3 g of a polymethylhydrosiloxane crosslinking agent using the high-speed centrifugal mixer at 3470 rpm for 5 minutes. As noted above, specimens were prepared by applying the two parts to a substrate via a dual-barrel syringe. Specimens were cured at 80 °C for one minute.

Comparative Example 1

Component A: A diluted Karstedt's catalyst solution was prepared by mixing 2.65 g of a complex of 2.2% platinum tetramethyldivinyl disiloxane in xylene with 97.35 g of low-molecular-weight (Mn between about 4,000 and about 6,000) vinyl-terminated polydimethylsiloxane solution for three hours.

Component B: A diluted vinyl acetate solution was prepared by mixing 2.35 g of vinyl acetate with 97.65 g of low-molecular-weight vinyl-terminated polydimethylsiloxane solution for three hours.

An attempt was made to prepare a liquid silicone rubber from a two-part kit. To form the first part of the kit, 97 g of a mixture of vinyl-terminated polydimethylsiloxane and fumed silica particles was combined with 3 g of the diluted Karstedt's catalyst solution of Component A using the high-speed centrifugal mixer at 3470 rpm for 5 minutes. To form the second part of the kit, 94 g of a mixture of vinyl-terminated polydimethylsiloxane and fumed silica particles was combined with 3 g of a polymethylhydrosiloxane crosslinking agent and 3 g of the diluted vinyl acetate solution of Component B using the high-speed centrifugal mixer at 3470 rpm for 5 minutes. Despite the platinum concentration in the first part of Comparative Example 1 being the same as that for the first part of Inventive Example 1, a specimen could not be formed. Upon exiting the syringe and prior to any heat exposure, the mixture of the first and second parts of Comparative Example 1 was unable to form a free-standing silicone film or any other structure or shape.

Inventive Example 2

An illustrative low-temperature-cured liquid silicone rubber with 5% triclosan was formed from a two-part kit in a similar fashion to Inventive Example 1. The first part of the kit was formed by combining 95 g of the mixture described for the first part of Inventive Example 1 with 5 g of triclosan using the high-speed centrifugal mixer at 3470 rpm for 5 minutes. The second part of the kit was formed by combining 95 g of the mixture described for the second part of Inventive Example 1 with 5 g of triclosan using the high-speed centrifugal mixer at 3470 rpm for 5 minutes. As with Inventive Example 1, the two parts were applied to a substrate via a dual-barrel syringe, and specimens were cured at 80 °C for one minute.

Inventive Example 3

An illustrative low-temperature-cured liquid silicone rubber with 10% triclosan was formed from a two-part kit in a similar fashion to Inventive Examples 1 and 2. The first part of the kit was formed by combining 90 g of the mixture described for the first part of Inventive Example 1 with 10 g of triclosan using the high-speed centrifugal mixer at 3470 rpm for 5 minutes. The second part of the kit was formed by combining 90 g of the mixture described for the second part of Inventive Example 1 with 10 g of triclosan using the high-speed centrifugal mixer at 3470 rpm for 5 minutes. As with Inventive Examples 1 and 2, the two parts were applied to a substrate via a dual-barrel syringe, and specimens were cured at 80 °C for one minute.

Inventive Example 4

An illustrative low-temperature-cured liquid silicone rubber with 33.3% triclosan was formed from a two-part kit in a similar fashion to Inventive Examples 1-3. The first part of the kit was formed by combining 66.7 g of the mixture described for the first part of Inventive Example 1 with 33.3 g of triclosan using the high-speed centrifugal mixer at 3470 rpm for 5

minutes. The second part of the kit was formed by combining 66.7 g of the mixture described for the second part of Inventive Example 1 with 33.3 g of triclosan using the high-speed centrifugal mixer at 3470 rpm for 5 minutes. As with Inventive Examples 1-3, the two parts were applied to a substrate via a dual-barrel syringe, and specimens were cured at 80 °C for one minute.

Comparative Example 2

A conventional liquid silicone rubber was formed from a two-part kit. First, a Karstedt's catalyst solution was prepared by mixing 2.5 g of a complex of 2.2% platinum tetramethyldivinyl disiloxane in xylene with 97.5 g of low-molecular-weight vinyl-terminated polydimethylsiloxane solution for one hour at ambient temperature. To form the first part of the kit, 97 g of a mixture of vinyl-terminated polydimethylsiloxane and fumed silica particles was combined with 3 g of the above-described Karstedt catalyst solution using the high-speed centrifugal mixer at 3470 rpm for 5 minutes. To form the second part of the kit, 96 g of a mixture of vinyl-terminated polydimethylsiloxane and fumed silica particles was combined with 3 g of a polymethylhydrosiloxane crosslinking agent and 1 g of ethynylcyclohexanol using the high-speed centrifugal mixer at 3470 rpm for 5 minutes. Specimens were prepared as described above, by applying the two parts to a substrate via a dual-barrel syringe; however, specimens were cured at 180 °C for one minute.

As shown below in Table 1, mechanical properties were tested for the specimens relating to Inventive Examples 1-4 and Comparative Example 2. Specifically, the specimens were tested for tensile strength, elongation at break, and hardness in accordance with the procedures described herein.

Table 1: Test Results for Mechanical Properties

| Specimen | Tensile Strength (kg/m (lb/in)) | Elongation at break (%) | Hardness (Shore M Durometer) |
|---|---|---|---|
| Inventive Example 1 | 6768 (379) | 351 | 33 |
| Inventive Example 2 | 5429 (304) | 370 | 35 |
| Inventive Example 3 | 4875 (273) | 291 | 38 |
| Inventive Example 4 | 3197 (179) | 150 | 42 |
| Comparative Example 2 | 6929 (388) | 329 | 35 |

The results shown in Table 1 indicate that the illustrative low-temperature-cured liquid silicone rubbers, with or without an API (e.g., triclosan), can provide mechanical properties that are comparable to, and in some cases better than, conventional liquid silicone rubbers, such that the illustrative low-temperature-cured liquid silicone rubbers exhibit sufficient mechanical properties to be able to formed into a desired geometry.

VII. Miscellaneous

It is understood that any one or more of the teachings, expressions, embodiments, examples, versions, etc. described herein may be combined with any one or more of the other teachings, expressions, embodiments, examples, versions etc. that are described herein. The above-described teachings, expressions, embodiments, examples, versions etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DA VINCI® system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK® bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

**1.** A low-temperature-curable liquid silicone rubber comprising:

(a) a polydimethylsiloxane;
(b) a crosslinking agent;
(c) a platinum tetramethyldivinyl disiloxane vinyl acetate catalyst; and
(d) a surface-treated silica filler.

**2.** The low-temperature-curable liquid silicone rubber of claim 1 comprising about 60% to about 90%, by weight, of the polydimethylsiloxane.

**3.** The low-temperature-curable liquid silicone rubber of any one of claims 1-2 comprising about 10% to about 40% of the surface-treated silica filler.

**4.** The low-temperature-curable liquid silicone rubber of any one of claims 1-3 being curable at a temperature of about 60 °C to about 100 °C, optionally being curable at a temperature of about 80 °C.

**5.** The low-temperature-curable liquid silicone rubber of any one of claims 1-4 being curable in about 2 minutes or less.

**6.** The low-temperature-curable liquid silicone rubber of any one of claims 1-5 being curable in about 1 minute or less.

**7.** The low-temperature-curable liquid silicone rubber of any one of claims 1-6 being curable in less than about 30 seconds.

**8.** The low-temperature-curable liquid silicone rubber of any one of claims 1-7 comprising about 0.050% to about 0.075%, by weight, of the platinum tetramethyldivinyl disiloxane vinyl acetate catalyst.

**9.** The low-temperature-curable liquid silicone rubber of any one of claims 1-8, wherein:

(a) the platinum tetramethyldivinyl disiloxane vinyl acetate catalyst registers a $^{195}Pt$ signal at about -5837 ppm on an NMR spectrum; and/or
(b) the platinum tetramethyldivinyl disiloxane vinyl acetate catalyst comprises less than 50% vinyl acetate, by weight of the platinum tetramethyldivinyl disiloxane vinyl acetate catalyst; and/or
(c) the platinum tetramethyldivinyl disiloxane vinyl acetate catalyst is formed from treating platinum tetramethyldivinyl disiloxane with a vinyl acetate for three or more hours to form platinum tetramethyldivinyl disiloxane vinyl acetate; wherein the platinum tetramethyldivinyl disiloxane and the vinyl acetate are provided in a mole ratio of 2 to 100 of vinyl acetate to platinum tetramethyldivinyl disiloxane; and/or
(d) the polydimethylsiloxane comprises vinyl-terminated polydimethylsiloxane; and/or
(e) the crosslinking agent comprises one or more of polymethylhydrosiloxane and polymethylhydro-co-polydimethylsiloxane; and/or
(f) the surface-treated silica filler comprises hexamethyldisilazane-treated silica.

**10.** The low-temperature-curable liquid silicone rubber of any one of claims 1-9, further comprising one or more active pharmaceutical ingredients (API), optionally wherein (a) the API comprises triclosan; and/or (b) the low-temperature-curable liquid silicone rubber comprises about 5% to about 35%, by weight, of the API.

**11.** A medical device comprising a low-temperature-cured liquid silicone rubber formed from the low-temperature-curable liquid silicone rubber of claim 10, wherein the API can transfer from the low-temperature-cured liquid silicone rubber to an intended recipient.

**12.** A compositional kit for forming a low-temperature-curable liquid silicone rubber, the kit comprising:

(a) a first composition comprising a mixture of vinyl-terminated polydimethylsiloxane, surface-treated silica particles, and a platinum tetramethyldivinyl disiloxane vinyl acetate catalyst solution; and
(b) a second composition comprising a mixture of vinyl-terminated polydimethylsiloxane, surface-treated silica particles, and a crosslinking agent.

**13.** The kit of claim 12, further comprising a dual-barrel syringe, wherein the first composition is disposed in a first barrel and the second composition is disposed in a second barrel; wherein the first and second compositions are provided in equal parts; and wherein the dual-barrel syringe is configured to simultaneously express the first and second compositions through a static mixer.

**14.** A catalyst for silicone cross-linking, the catalyst formed from treating platinum tetramethyldivinyl disiloxane with a vinyl acetate for three or more hours to form platinum tetramethyldivinyl disiloxane vinyl acetate; wherein the platinum tetramethyldivinyl disiloxane and the vinyl acetate are provided in a mole ratio of 2 to 100 of vinyl acetate to platinum tetramethyldivinyl disiloxane.

**15.** The catalyst of claim 14, wherein (a) the vinyl acetate comprises less than 50%, by weight, of the catalyst; and/or (b) the platinum tetramethyldivinyl disiloxane is treated with the vinyl acetate for four or more hours.

## Patentansprüche

**1.** Bei niedriger Temperatur härtbarer flüssiger Silikonkautschuk, umfassend:

(a) ein Polydimethylsiloxan;
(b) ein Vernetzungsmittel;
(c) einen Platin-Tetramethyldivinyldisiloxan-Vinylacetat-Katalysator; und
(d) einen oberflächenbehandelten Siliciumdioxid-Füllstoff.

**2.** Bei niedriger Temperatur härtbarer flüssiger Silikonkautschuk nach Anspruch 1, umfassend etwa 60 Gew.-% bis etwa 90 Gew.-% des Polydimethylsiloxans.

**3.** Bei niedriger Temperatur härtbarer flüssiger Silikonkautschuk nach einem der Ansprüche 1 bis 2, umfassend etwa 10 % bis etwa 40 % des oberflächenbehandelten Siliciumdioxid-Füllstoffs.

**4.** Bei niedriger Temperatur härtbarer flüssiger Silikonkautschuk nach einem der Ansprüche 1 bis 3, der bei einer Temperatur von etwa 60 °C bis etwa 100 °C härtbar ist, gegebenenfalls bei einer Temperatur von etwa 80 °C härtbar ist.

**5.** Bei niedriger Temperatur härtbarer flüssiger Silikonkautschuk nach einem der Ansprüche 1 bis 4, der in etwa 2 Minuten oder weniger härtbar ist.

**6.** Bei niedriger Temperatur härtbarer flüssiger Silikonkautschuk nach einem der Ansprüche 1 bis 5, der in etwa 1 Minute oder weniger härtbar ist.

**7.** Bei niedriger Temperatur härtbarer flüssiger Silikonkautschuk nach einem der Ansprüche 1 bis 6, der in weniger als etwa 30 Sekunden härtbar ist.

**8.** Bei niedriger Temperatur härtbarer flüssiger Silikonkautschuk nach einem der Ansprüche 1 bis 7, umfassend etwa

0,050 Gew.-% bis etwa 0,075 Gew.-% des Platin-Tetramethyldivinyldisiloxan-Vinylacetat-Katalysators.

**9.** Bei niedriger Temperatur härtbarer flüssiger Silikonkautschuk nach einem der Ansprüche 1 bis 8, wobei:

(a) der Platin-Tetramethyldivinyldisiloxan-Vinylacetat-Katalysator ein $^{195}$Pt-Signal bei etwa -5837 ppm in einem NMR-Spektrum registriert; und/oder
(b) der Platin-Tetramethyldivinyldisiloxan-Vinylacetat-Katalysator weniger als 50 % Vinylacetat umfasst, bezogen auf das Gewicht des Platin-Tetramethyldivinyldisiloxan-Vinylacetat-Katalysators; und/oder
(c) der Platin-Tetramethyldivinyldisiloxan-Vinylacetat-Katalysator durch Behandeln von Platin-Tetramethyldivinyldisiloxan mit einem Vinylacetat für drei oder mehr Stunden zur Bildung von Platin-Tetramethyldivinyldisiloxan-Vinylacetat gebildet wird; wobei das Platin-Tetramethyldivinyldisiloxan und das Vinylacetat in einem Molverhältnis von 2 zu 100 von Vinylacetat zu Platin-Tetramethyldivinyldisiloxan bereitgestellt sind; und/oder
(d) das Polydimethylsiloxan vinylterminiertes Polydimethylsiloxan umfasst; und/oder
(e) das Vernetzungsmittel eines oder mehrere von Polymethylhydrosiloxan und Polymethylhydro-co-polydimethylsiloxan umfasst; und/oder
(f) der oberflächenbehandelte Siliciumdioxid-Füllstoff mit Hexamethyldisilazan behandeltes Siliciumdioxid umfasst.

**10.** Bei niedriger Temperatur härtbarer flüssiger Silikonkautschuk nach einem der Ansprüche 1 bis 9, ferner einen oder mehrere pharmazeutische Wirkstoffe (API) umfassend, gegebenenfalls wobei (a) der API Triclosan umfasst; und/oder (b) der bei niedriger Temperatur härtbare flüssige Silikonkautschuk etwa 5 Gew.-% bis etwa 35 Gew.-% des API umfasst.

**11.** Medizinische Vorrichtung, umfassend einen bei niedriger Temperatur gehärteten flüssigen Silikonkautschuk, der aus dem bei niedriger Temperatur härtbaren flüssigen Silikonkautschuk nach Anspruch 10 gebildet ist, wobei der API von dem bei niedriger Temperatur gehärteten flüssigen Silikonkautschuk zu einem vorgesehenen Empfänger übertragen werden kann.

**12.** Zusammensetzungskit zur Bildung eines bei niedriger Temperatur härtbaren flüssigen Silikonkautschuks, wobei das Kit umfasst:

(a) eine erste Zusammensetzung, umfassend eine Mischung aus vinylterminiertem Polydimethylsiloxan, oberflächenbehandelten Siliciumdioxidpartikeln und einer Platin-Tetramethyldivinyldisiloxan-Vinylacetat-Katalysator-Lösung; und
(b) eine zweite Zusammensetzung, umfassend eine Mischung aus vinylterminiertem Polydimethylsiloxan, oberflächenbehandelten Siliciumdioxidpartikeln und einem Vernetzungsmittel.

**13.** Kit nach Anspruch 12, ferner umfassend eine Doppelkolbenspritze, wobei die erste Zusammensetzung in einem ersten Kolben angeordnet ist und die zweite Zusammensetzung in einem zweiten Kolben angeordnet ist; wobei die erste und zweite Zusammensetzung in gleichen Teilen bereitgestellt sind; und wobei die Doppelkolbenspritze konfiguriert ist, um die erste und zweite Zusammensetzung gleichzeitig durch einen Statikmischer auszudrücken.

**14.** Katalysator für die Silikonvernetzung, wobei der Katalysator durch Behandlung von Platin-Tetramethyldivinyldisiloxan mit einem Vinylacetat für drei oder mehr Stunden zur Bildung von Platin-Tetramethyldivinyldisiloxan-Vinylacetat gebildet wird; wobei das Platin-Tetramethyldivinyldisiloxan und das Vinylacetat in einem Molverhältnis von 2 zu 100 von Vinylacetat zu Platin-Tetramethyldivinyldisiloxan bereitgestellt sind.

**15.** Katalysator nach Anspruch 14, wobei (a) das Vinylacetat weniger als 50 Gew.-% des Katalysators umfasst; und/oder (b) das Platin-Tetramethyldivinyldisiloxan vier oder mehr Stunden lang mit dem Vinylacetat behandelt wird.

## Revendications

**1.** Caoutchouc de silicone liquide durcissable à basse température comprenant :

(a) un polydiméthylsiloxane ;
(b) un agent de réticulation ;
(c) un catalyseur platine tétraméthyldivinyl-disiloxane acétate de vinyle ; et

(d) une charge de silice traitée en surface.

**2.** Caoutchouc de silicone liquide durcissable à basse température selon la revendication 1 comprenant environ 60 % à environ 90 %, en poids, du polydiméthylsiloxane.

**3.** Caoutchouc de silicone liquide durcissable à basse température selon l'une quelconque des revendications 1 à 2 comprenant environ 10 % à environ 40 % de la charge de silice traitée en surface.

**4.** Caoutchouc de silicone liquide durcissable à basse température selon l'une quelconque des revendications 1 à 3 pouvant durcir à une température d'environ 60 °C à environ 100 °C, facultativement pouvant durcir à une température d'environ 80 °C.

**5.** Caoutchouc de silicone liquide durcissable à basse température selon l'une quelconque des revendications 1 à 4 pouvant durcir en environ 2 minutes ou moins.

**6.** Caoutchouc de silicone liquide durcissable à basse température selon l'une quelconque des revendications 1 à 5 pouvant durcir en environ 1 minute ou moins.

**7.** Caoutchouc de silicone liquide durcissable à basse température selon l'une quelconque des revendications 1 à 6 pouvant durcir en moins d'environ 30 secondes.

**8.** Caoutchouc de silicone liquide durcissable à basse température selon l'une quelconque des revendications 1 à 7 comprenant environ 0,050 % à environ 0,075 %, en poids, du catalyseur platine tétraméthyldivinyl-disiloxane acétate de vinyle.

**9.** Caoutchouc de silicone liquide durcissable à basse température selon l'une quelconque des revendications 1 à 8, dans lequel :

(a) le catalyseur platine tétraméthyldivinyl-disiloxane acétate de vinyle enregistre un signal $^{195}Pt$ à environ -5837 ppm sur un spectre RMN ; et/ou
(b) le catalyseur platine tétraméthyldivinyl-disiloxane acétate de vinyle comprend moins de 50 % d'acétate de vinyle, en poids du catalyseur platine tétraméthyldivinyl-disiloxane acétate de vinyle ; et/ou
(c) le catalyseur platine tétraméthyldivinyl-disiloxane acétate de vinyle est formé par un traitement de platine tétraméthyldivinyl-disiloxane avec un acétate de vinyle pendant trois heures ou plus pour former du platine tétraméthyldivinyl-disiloxane acétate de vinyle ; dans lequel le platine tétraméthyldivinyl-disiloxane et l'acétate de vinyle sont fournis dans un rapport molaire de 2 à 100 de l'acétate de vinyle au platine tétraméthyldivinyl-disiloxane ; et/ou
(d) le polydiméthylsiloxane comprend du polydiméthylsiloxane à terminaison vinyle ; et/ou
(e) l'agent de réticulation comprend un ou plusieurs parmi polyméthylhydrosiloxane et polyméthylhydro-co-polydiméthylsiloxane ; et/ou
(f) la charge de silice traitée en surface comprend de la silice traitée par hexaméthyldisilazane.

**10.** Caoutchouc de silicone liquide durcissable à basse température selon l'une quelconque des revendications 1 à 9, comprenant en outre un ou plusieurs ingrédients pharmaceutiques actifs (API), facultativement dans lequel (a) l'API comprend du triclosan ; et/ou (b) le caoutchouc de silicone liquide durcissable à basse température comprend environ 5 % à environ 35 %, en poids, de l'API.

**11.** Dispositif médical comprenant un caoutchouc de silicone liquide durci à basse température formé à partir du caoutchouc de silicone liquide durcissable à basse température selon la revendication 10, dans lequel l'API peut se transférer du caoutchouc de silicone liquide durci à basse température vers un destinataire prévu.

**12.** Trousse de compositions permettant de former un caoutchouc de silicone liquide durcissable à basse température, la trousse comprenant :

(a) une première composition comprenant un mélange de polydiméthylsiloxane à terminaison vinyle, de particules de silice traitées en surface, et d'une solution de catalyseur platine tétraméthyldivinyl-disiloxane acétate de vinyle ; et
(b) une seconde composition comprenant un mélange de polydiméthylsiloxane à terminaison vinyle, de

particules de silice traitées en surface, et d'un agent de réticulation.

**13.** Trousse selon la revendication 12, comprenant en outre une seringue à double cylindre, dans laquelle la première composition est disposée dans un premier cylindre et la seconde composition est disposée dans un second cylindre ; dans laquelle les première et seconde compositions sont fournies en parts égales ; et dans laquelle la seringue à double cylindre est conçue pour expulser simultanément les première et seconde compositions à travers un mélangeur statique.

**14.** Catalyseur destiné à la réticulation de silicone, le catalyseur étant formé par le traitement de platine tétraméthyldivinyl-disiloxane avec un acétate de vinyle pendant trois heures ou plus pour former du platine tétraméthyldivinyl-disiloxane acétate de vinyle ; dans lequel le platine tétraméthyldivinyl-disiloxane et l'acétate de vinyle sont fournis dans un rapport molaire de 2 à 100 de l'acétate de vinyle au platine tétraméthyldivinyl-disiloxane.

**15.** Catalyseur selon la revendication 14, dans lequel (a) l'acétate de vinyle constitue moins de 50 %, en poids, du catalyseur ; et/ou (b) le platine tétraméthyldivinyl-disiloxane est traité avec l'acétate de vinyle pendant quatre heures ou plus.

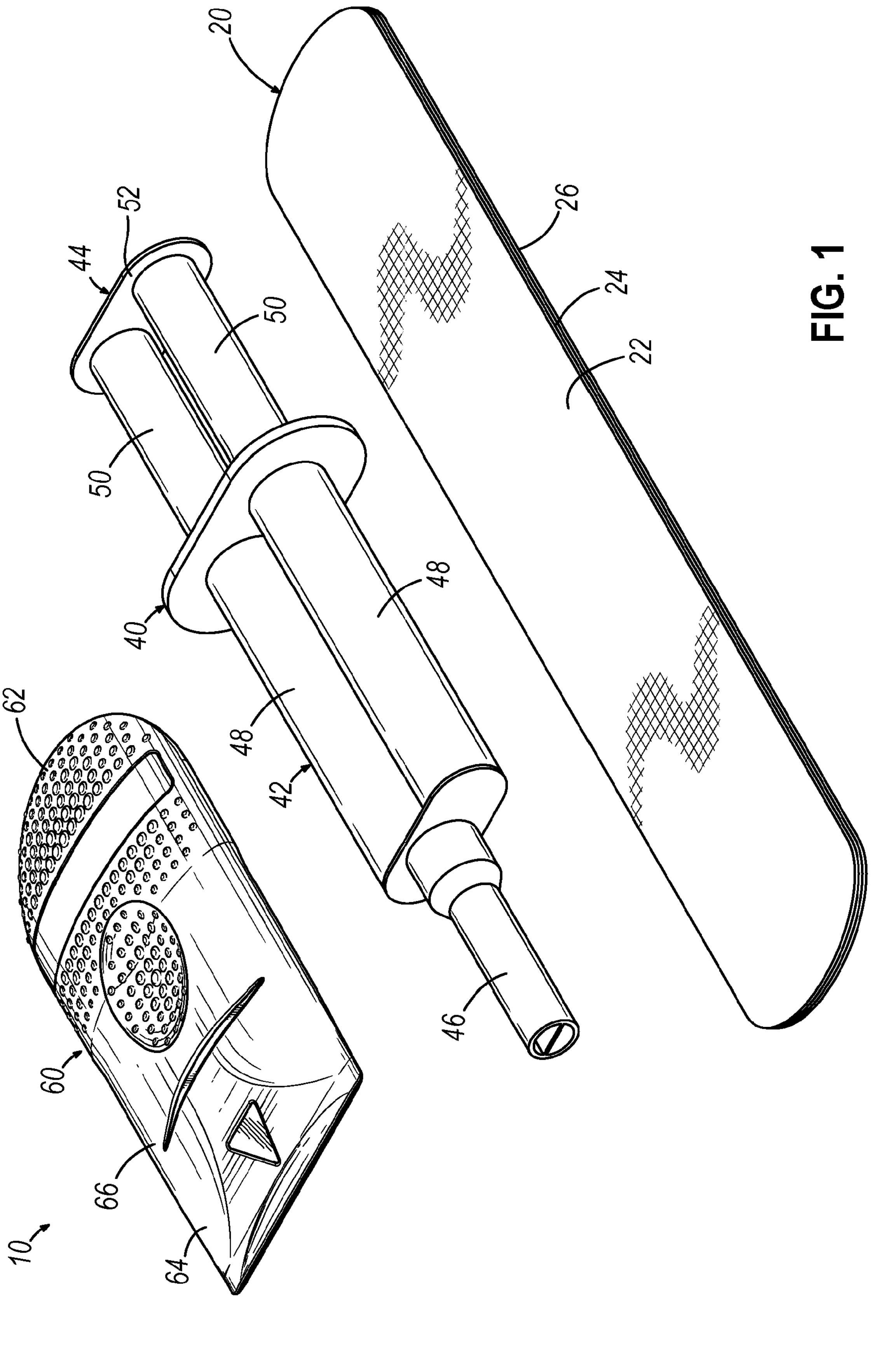


FIG. 1

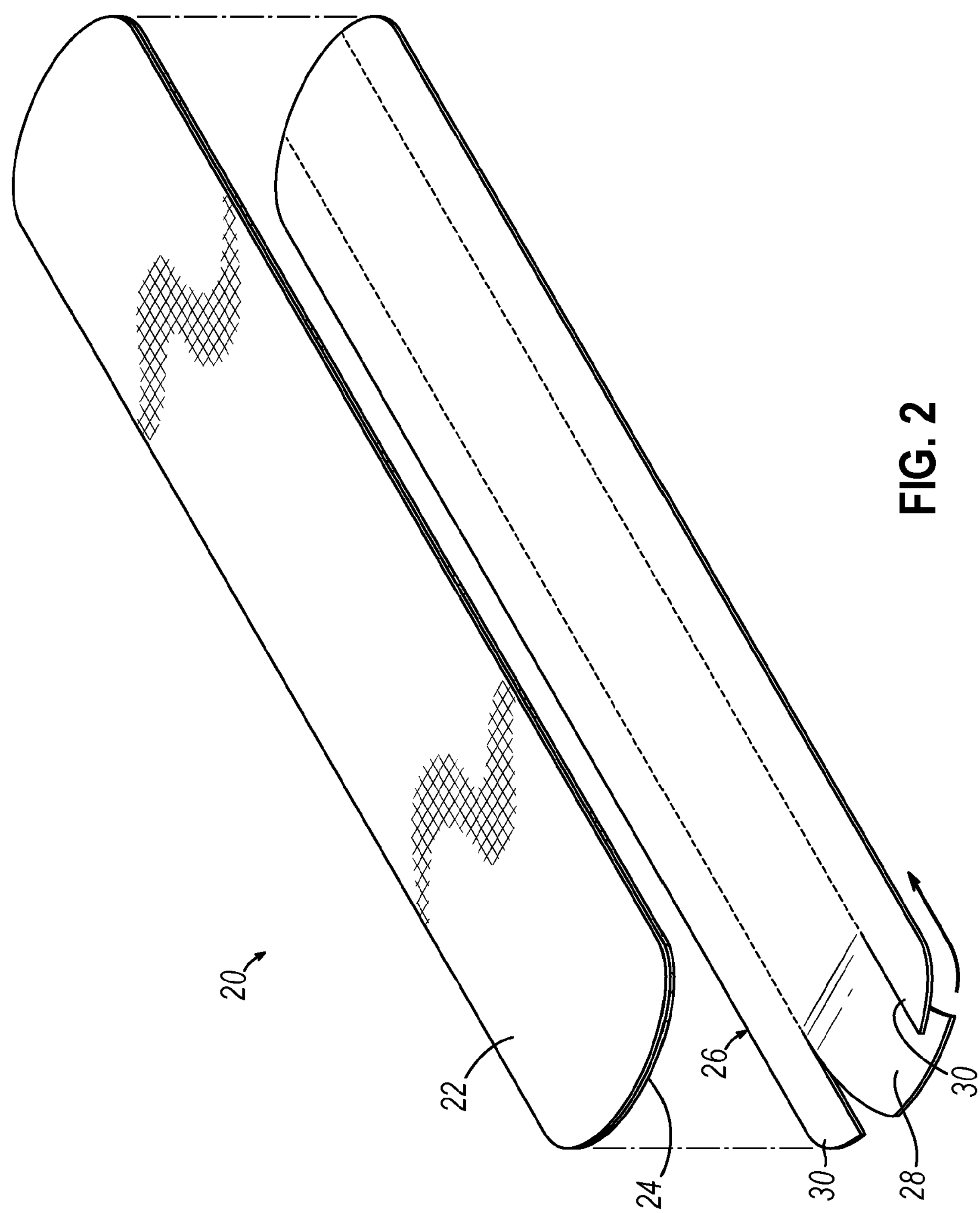


FIG. 2

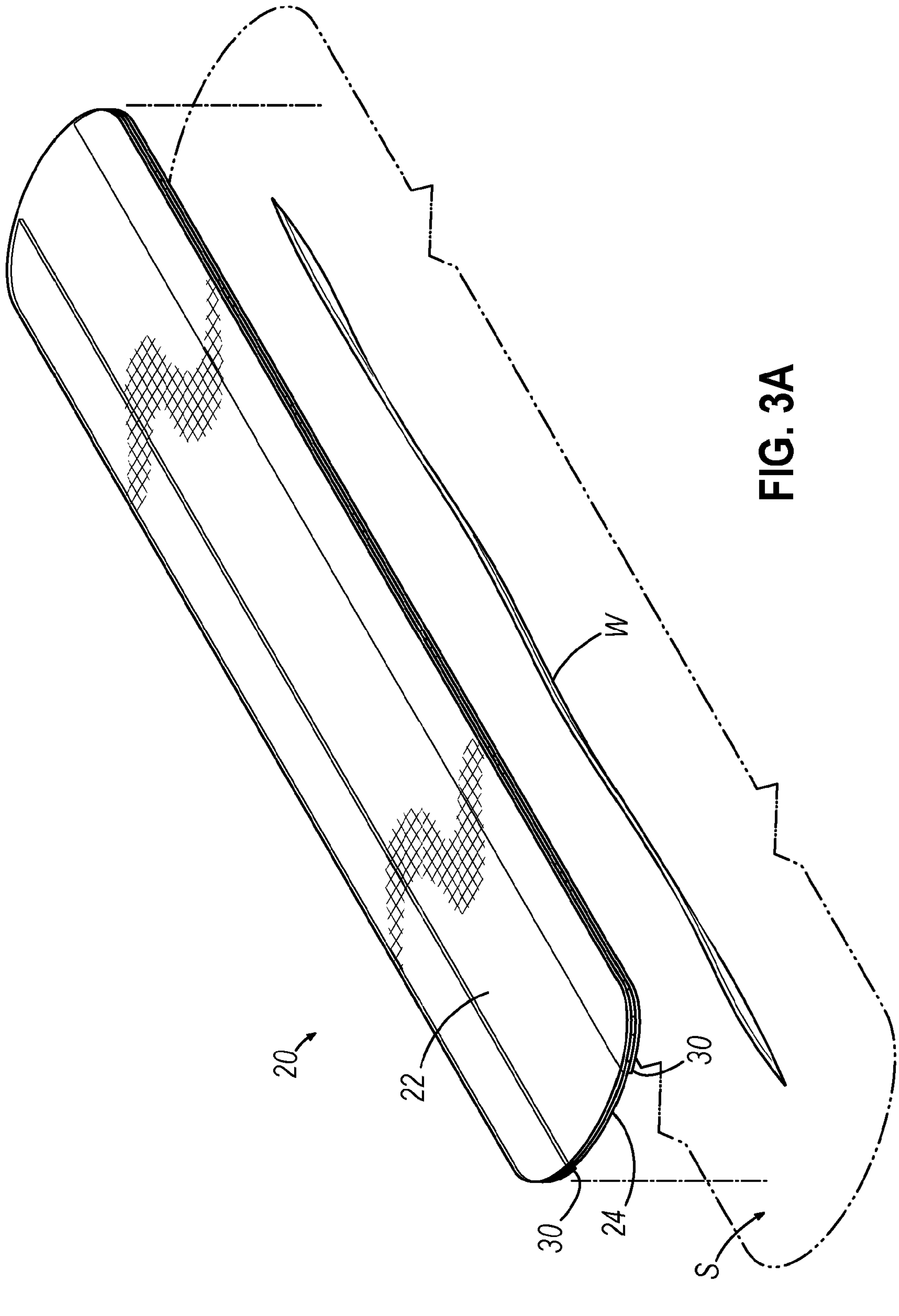


FIG. 3A

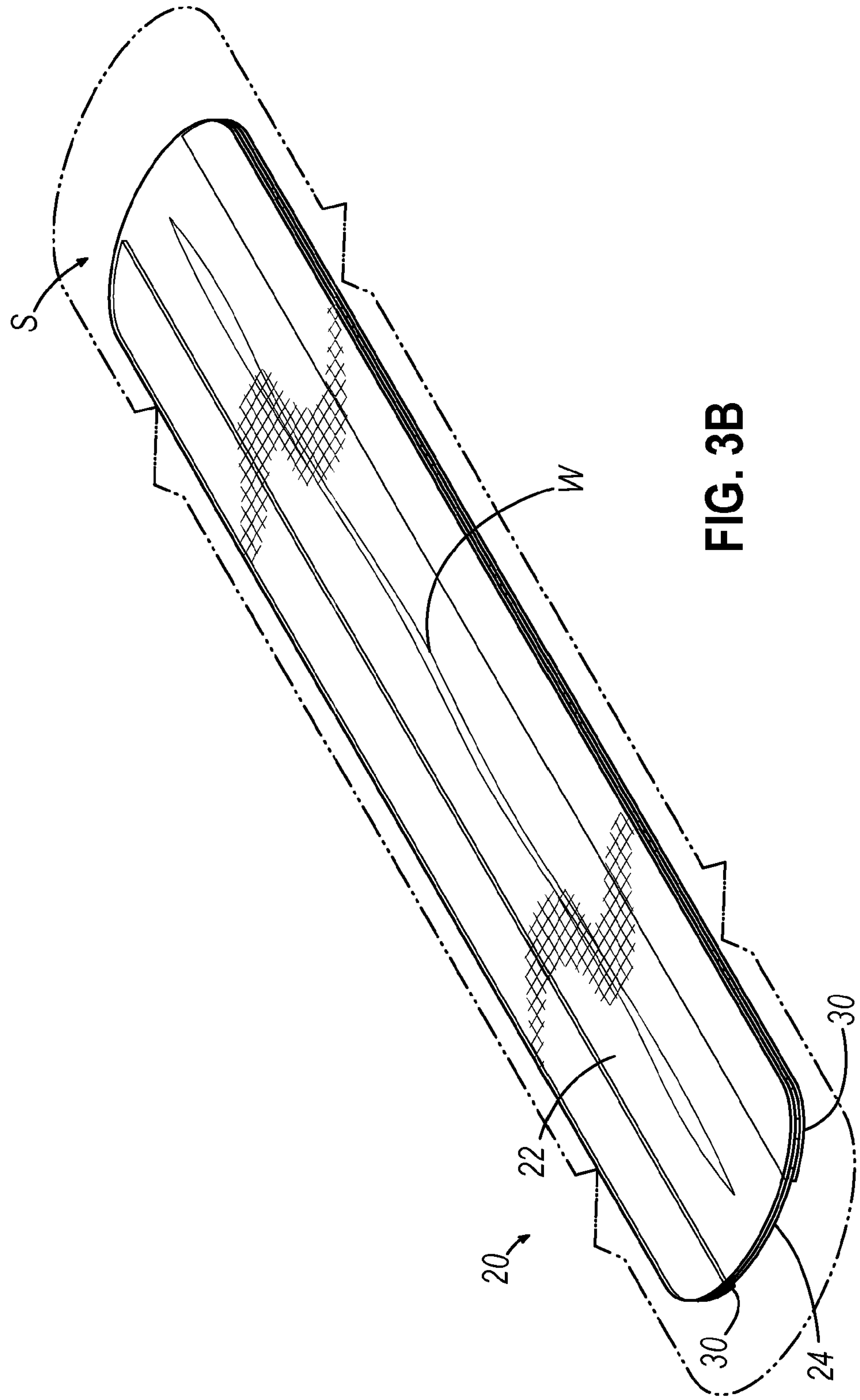


FIG. 3B

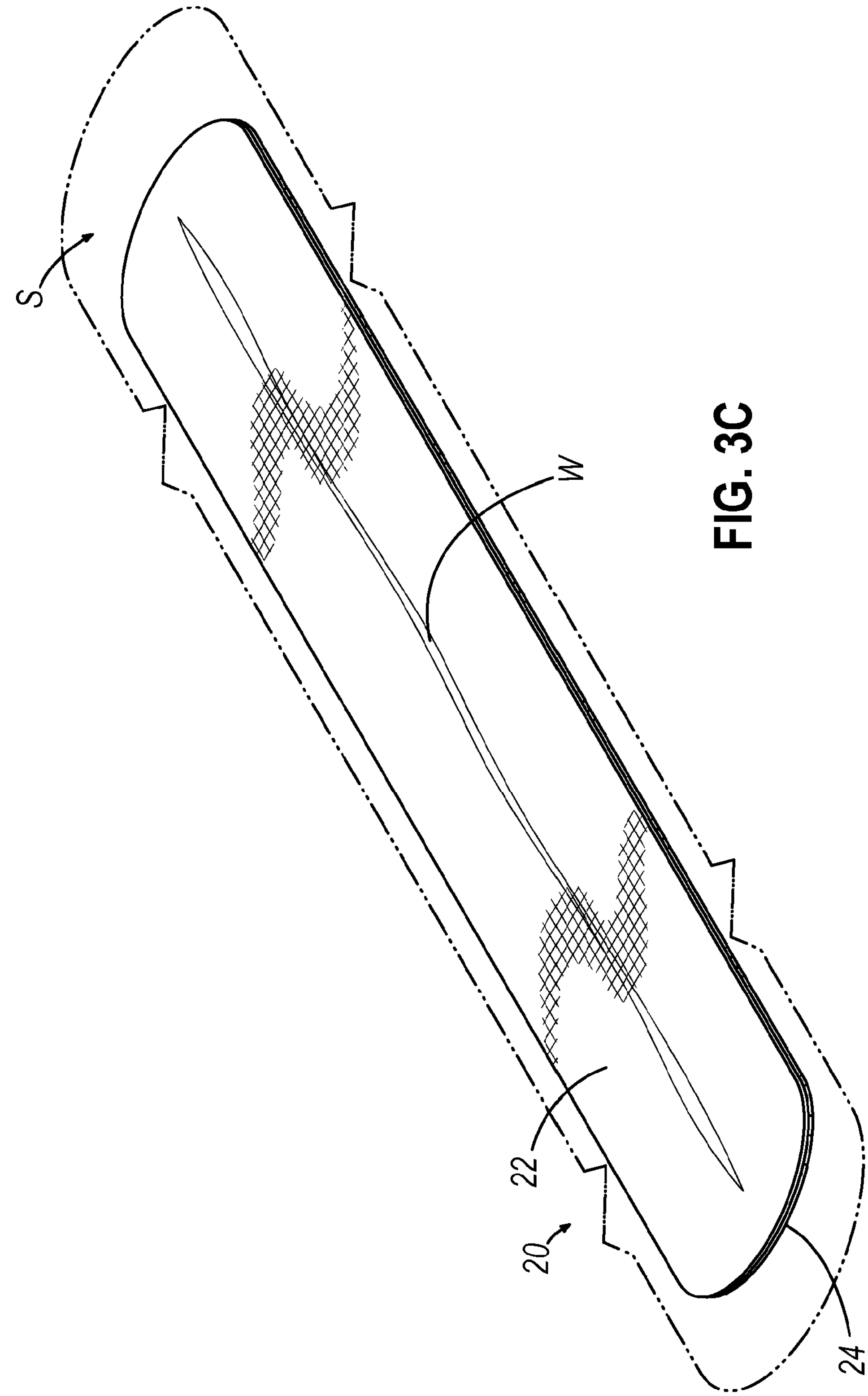


FIG. 3C

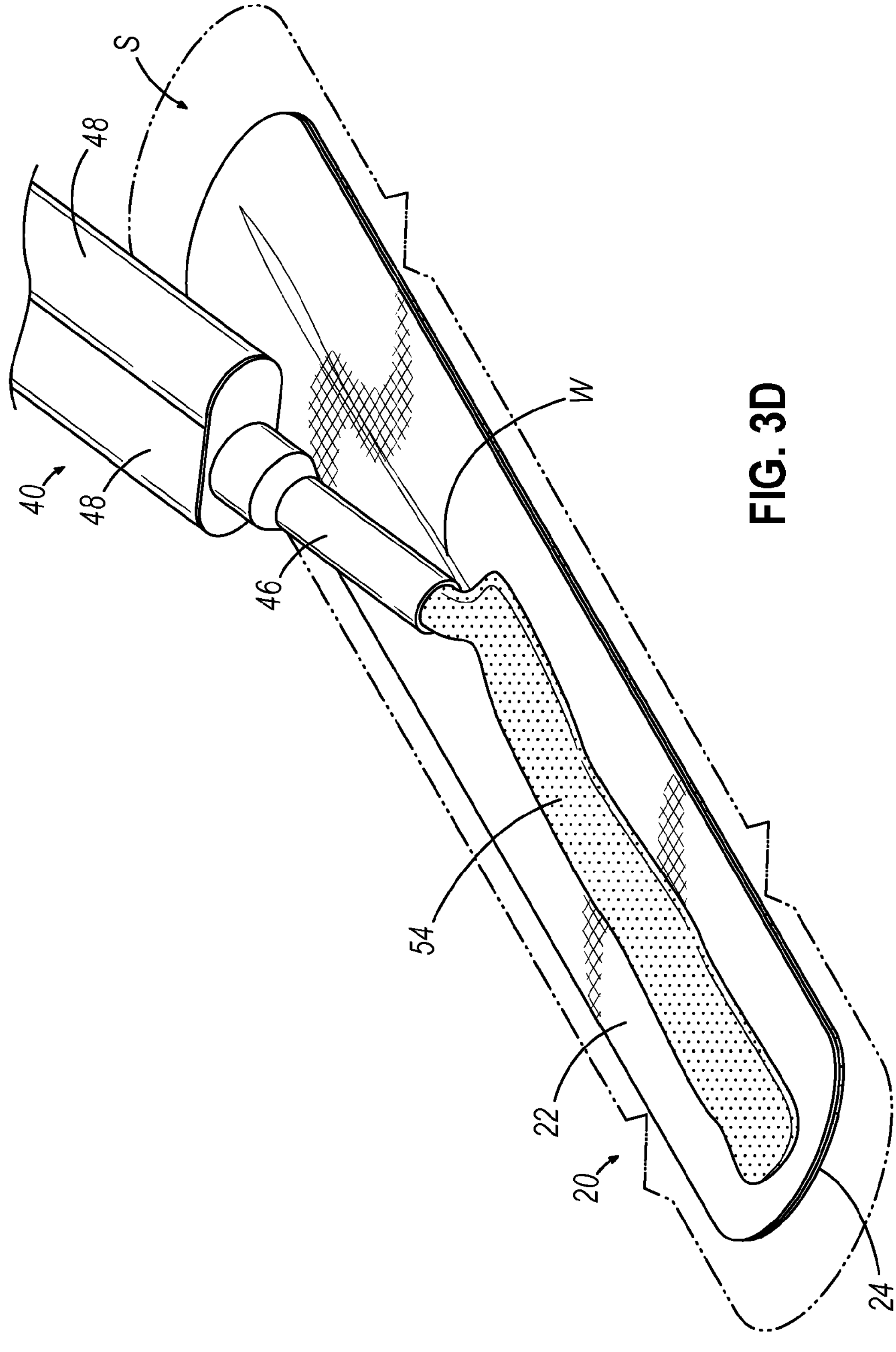


FIG. 3D

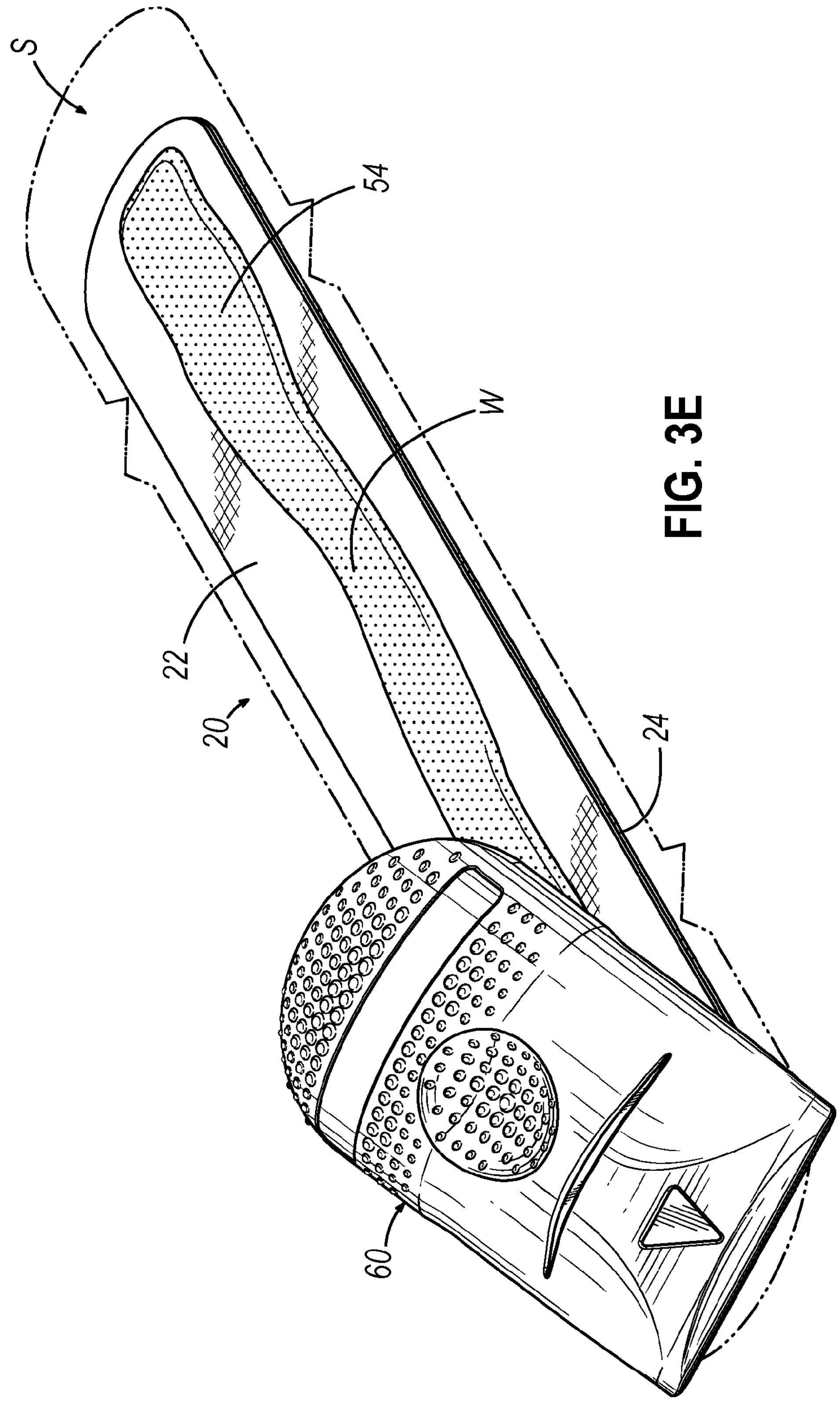


FIG. 3E

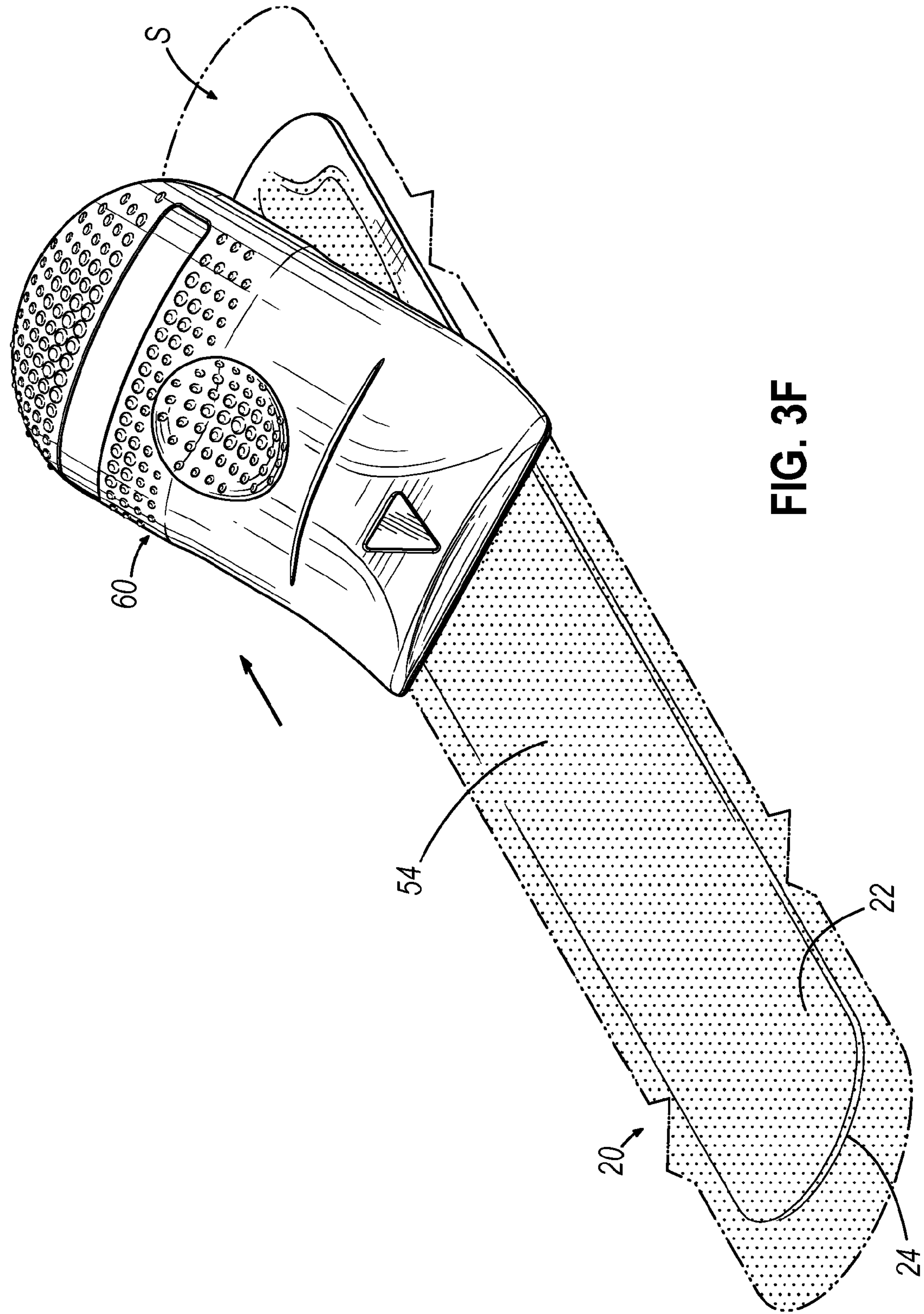


FIG. 3F

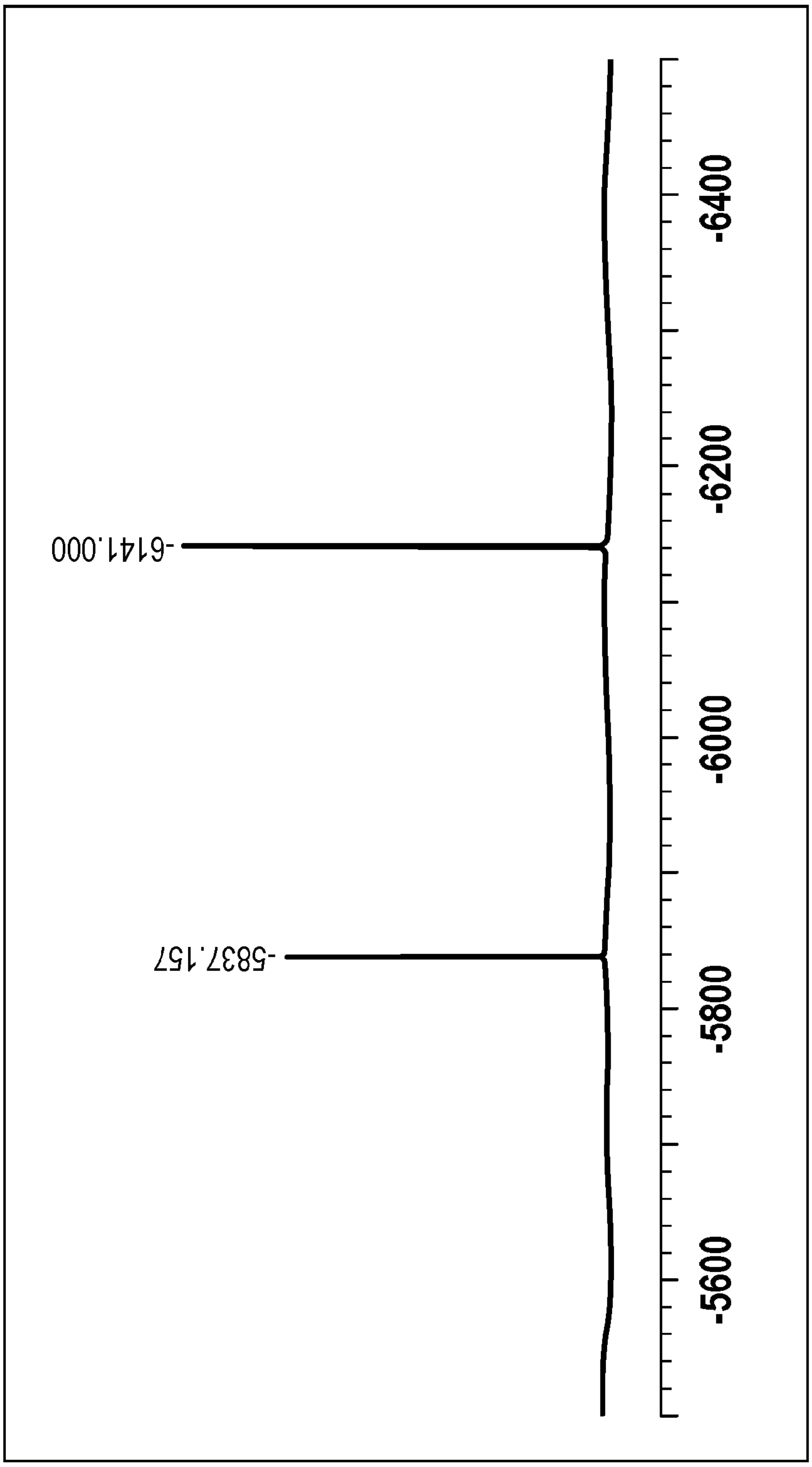


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20210369258 A
- US 20210371190 A
- US 20210371658 A
- US 20210369639 A
- US 20210369276 A
- US 66795022
- US 99199222
- US 17991945 B
- US 99195022


### Non-patent literature cited in the description


- **A. J. BARRY**. *Journal of Applied Physics*, 1946, vol. 17, 1020